# EUROPÄISCHE PATENTANMELDUNG

(11) **EP 0 763 597 A2**
(43) Veröffentlichungstag der Anmeldung: **19.03.1997**
(21) Anmeldenummer: 96114413.6
(22) Anmeldetag: 09.09.1996
(51) Int. Cl.: C12N 15/12, A61K 38/17, G01N 33/68, G01N 33/543, C12N 5/10, C12N 15/86, C12N 15/62

(54) **Verwendung von Cytohesin-PH-Peptiden zur Beeinflussung der Adhäsionsfähigkeit von Integrinen**

(30) Priorität: 14.09.1995 DE 19534120
(71) Anmelder: HOECHST AKTIENGESELLSCHAFT, 65926 Frankfurt am Main (DE)
(72) Erfinder: Kolanus, Waldemar, Dr., 81245 München (DE); Ostner, Britta, DB., 81675 München (DE)

(57) **Zusammenfassung**

Die Erfindung betrifft die Verwendung von Cytohesin-PH-Peptiden zur Regulation der T-Lymphozyten-Aktivierung sowie die Verwendung der Cytohesin-kodierenden DNA zur Expression des genannten Peptids, sowie Testkits und Testverfahren zum Screening nach Substanzen, die die Integrin vermittelte Zelladhäsion beeinflussen.

## Beschreibung

Die T-Lymphozyten-Aktivierung wird durch eine koordinierte Bindung von Adhäsionsmolekülrezeptoren und Signalrezeptoren erreicht, welche auf der Oberfläche von T-Zellen dann exprimiert werden, wenn diese Rezeptoren an ihre Gegenrezeptoren auf der Antigen-präsentierenden Zelle binden. Neben den bei der Leukozyten-Aktivierung immer beteiligten T-Zell-Rezeptoren (TCR's) und MHC (major histocompatibility class) Klasse I oder II Proteinen sind noch verschiedene Arten von Corezeptoren nötig z.B. Integrine, CD2, CD4 oder CD8-Moleküle. Die funktionelle Interaktion zwischen den TCR und den T-Lymphozyten-Corezeptoren ist dynamischer Natur, d.h. erst die Bindung eines TCR an sein Zielmolekül bewirkt eine verstärkte Bindung der Corezeptoren an ihre Gegenrezeptoren.

Die Integrine sind eine große Familie von Zelloberflächenmolekülen. Es sind Heterodimere, die aus Paaren nicht-disulfidverknüpfter α- und β-Ketten bestehen. Da es mehrere verschiedene α- und β-Ketten gibt, wird durch unterschiedliche Kombination der α- und β-Ketten eine unterschiedliche Ligandenspezifität erreicht. Die Integrine sind sowohl bei der direkten Zell-Zell-Interaktion als auch bei der Bindung von Zellen an die extra-zelluläre Matrix beteiligt.

Integrine, die auf nicht-aktivierten Lymphozyten vorkommen, befinden sich in einem sogenannten "low-avidity"-Zustand, der durch eine T-Zell-Aktivierung sehr schnell in einen transienten sogenannten "high-avidity"-Zustand umgewandelt wird. Der Mechanismus dieses sogenannten "inside-out signaling" ist bis heute ungeklärt (Collins et al. Curr. Opinion Imm. 6:385-393, 1994). Es existieren lediglich spekulative Vorstellungen über einen möglichen Mechanismus. Nach dem Affinitäts-Modulations-Modell kommt es bei einer T-Zell-Aktivierung zu einer Konformationsänderung bei den Integrinen, welche erst bewirkt, daß die hochaffine Ligandenbindungsstelle dem Liganden zugänglich wird. Als mögliche molekulare Ereignisse, welche die Konformationsänderung bewirken, werden z.Z. eine kovalent Modifizierung (z.B. Phosphorylierung) oder die Bindung von Aktivator bzw. Repressor-Molekülen an die cytoplasmatische Domäne der Integrin-β-Untereinheit diskutiert, ohne jedoch experimentelle Belege für einen bestimmten Mechanismus zu haben (Diamond and Springer, Curr. Biol. 4: 506-517, 1994).

Das hsec7hom (human SEC7 homolog) Protein wird hauptsächlich in natürlichen Killer-Zellen und cytotoxischen T-Zellen exprimiert. Entgegen der ursprünglichen Annahme, dieses Protein wäre das menschliche Homologe des SEC7 Proteins aus S. cerevisiae, ist aufgrund der stark unterschiedlichen Molekulargewichte von SEC7 und hsec7hom und der nur in einem relativ kurzen Abschnitt auftretenden Ähnlichkeit beider Proteine sowie einer sehr spezifischen Expression des hsec7hom (Liu und Pohaidak, Biochimica et Biophysica Acta 1132: 75-78, 1992) anzunehmen, daß hsec7hom nicht der SEC7 Proteinfamile angehört. Aus diesem Grunde nennen wir dieses Protein ab jetzt Cytohesin-1. Cytohesin-1 enthält zwei Regionen, die homolog zu Domänen anderer Proteine sind:
1. SEC7-Domäne, sie enthält ungefähr 200 Aminosäuren und ist erst von wenigen anderen Proteinen bekannt. Eines dieser Proteine ist das SEC7-Protein; es spielt eine Rolle bei der Sekretion in Hefen. Ein anderes Protein ist das erst kürzlich gefundene EMB30; es ist bei der Embryogenese von Arabidopsis involviert (Shevell et al., Cell 77: 1051-1062, 1994).
2. PH-Domäne (Pleckstrin Homologie-Domäne), sie ist ungefähr 100 Aminosäuren lang und in einer Reihe von Proteinen gefunden worden, von denen viele eine Rolle bei der Signaltransduktion spielen. Die dreidimensionale Struktur einiger PH-Domänen konnte kürzlich aufgeklärt werden. Es zeigte sich, daß sie als Ligandenbindungsdomänen fungieren können (Tsukada et al., Proc. Natl. Acad. Sci USA 91: 11256-60, 1994). Obwohl gezeigt werden konnte, daß die heterotrimeren G-Proteine mit PH-domänen interagieren können, konnte noch keine genaue physiologische Funktion für PH-Domänen ermittelt werden (Birney, TIBS 19: 349-353, 1994).

Der vorliegenden Erfindung lag die Aufgabe zugrunde, Proteine zu finden,die mit der cytoplasmatischen Domäne der β2-Untereinheit der Integrine (β2cyt) direkt und funktionell interagieren, um so die Möglichkeit zu erhalten, in das physiologische Geschehen bei allen biologischen Prozessen, bei denen die Modulation der "avidity" eine Rolle spielt, einzugreifen. Solche biologischen Prozesse sind z.B. die Wundheilung, die Entwicklung von Organen oder der große Bereich der Funktionen des Immunsystems.

Die Aufgabe wurde gelöst durch die Verwendung des "Two hybrid systems", auch "Interaction Trap" genannt (Gyuris et al., Cell 75: 791-803, 1993; Fields und Sternglanz, Trends in Genetics 10: 286-92, 1994). Dazu wurde die gesamte cytoplasmatische Domäne der β2-Integrin-Untereinheit (β2cyt) genau wie in der Literatur beschrieben (Kishimoto et al., Cell. 48: 681-690, 1987) an eine lex A-Bindungsdomäne fusioniert, um als "Köder" zu dienen. Da β2-Integrine spezifisch in Zellen hämatopoietischen Ursprungs exprimiert werden, wurde eine Hefe-Expressionsbank mit der cDNA aus Jurkat-Zellen (erhalten aus T-Zell-Tumoren) für das Screening benutzt.

Es wurde nun eine cDNA (cts 18.1) identifiziert, die große Ähnlichkeit zur vorbekannten B2-1 cDNA (Liu und Pohajdak) hat, welche für das oben beschriebene Cytohesin-1 kodiert, dessen Funktion noch unbekannt ist.

Das Genprodukt der cDNA cts 18.1 wird als Cytohesin-2 bezeichnet. Aufgrund der großen Ähnlichkeit von Cytohesin-1 und Cytohesin-2 (88 % Identität, 9 % konservierte Aminosäureaustausche) wird eine ähnliche oder gleiche Funktion beider Proteine angenommen. Aufgrund der Ähnlichkeit der cDNA's B2-1 und cts 18.1 ist auch eine Hybridisierung beider Moleküle unter stringenten Bedingungen nach dem Fachmann seit langem bekannten Verfahren möglich.

Überraschenderweise wurde nun gefunden, daß sich ein Peptid mit der Aminosäuresequenz der PH-Domäne von Cytohesin-1 oder Cytohesin-2, insbesondere Cytohesin-1 gemäß Tabelle 2, zur Regulation der T-Lymphozyten-Aktivierung verwenden läßt. Das genannte Peptid wird als Cytohesin-PH-Peptid bezeichnet.

Ein Gegenstand der Erfindung ist demgemäß die Verwendung eines Cytohesin-PH-Peptids, insbesondere gemäß Tabelle 2, besonders bevorzugt gemäß Tabelle 2, Aminosäureposition 258 bis 398, oder Teile davon, zur Regulation der T-Lymphozyten-Aktivierung.

Ein weiterer Gegenstand der Erfindung ist die Verwendung einer DNA, kodierend für ein Cytohesin-PH-Peptid, insbesondere gemäß Tabelle 2, besonders bevorzugt gemäß Tabelle 2, Nukleotidposition 841 bis 1263, oder Teile davon, zur Expression desselben Peptids.

Ein weiterer Gegenstand der Erfindung ist die Verwendung einer DNA, dadurch gekennzeichnet, daß deren Sequenz in bezug auf die Sequenz, der im vorigen Absatz erwähnten DNA entsprechend dem Wesen des genetischen Codes degeneriert ist.

Ein weiterer Gegenstand der Erfindung ist die Verwendung einer DNA, dadurch gekennzeichnet, daß sie unter stringenten Bedingungen mit der DNA gemäß Tabelle 2 hybridisiert.

Ein weiterer Gegenstand der Erfindung ist die Verwendung eines Vektors, enthaltend eine oben beschriebene DNA, zur Expression eines Cytohesin-PH-Peptids.

Ein weiterer Gegenstand der Erfindung ist die Verwendung einer Wirtszelle enthaltend einen der oben beschriebenen Vektoren.

Desweiteren sind Gegenstände der Erfindung die Verwendung eines oben beschriebenen Cytohesin-PH-Peptids zur Beeinflussung von Entzündungen, zur Verbesserung der Wundheilung, zur Suppression des Immunsystems, insbesondere bei Organtransplantationen, zur Verhinderung der Metastasierung bei hämatopoietischen Tumoren und zur Behandlung von Arteriosklerose.

Ein weiterer Gegenstand der Erfindung ist ein Arzneimittel enthaltend ein Cytohesin-PH-Peptid und einen physiologisch annehmbaren Träger sowie gegebenenfalls geeignete Zusatz- und/oder Hilfsstoffe.

Gemäß einem anderen Aspekt der vorliegenden Erfindung werden Test-Kits vorgeschlagen, die ein Cytohesin-PH-Peptid umfassen oder ein Cytohesin-PH-Peptid. Die Test-Kits können unter anderem für das Wirkstoff-Screening verwendet werden.

Gemäß einem weiteren Aspekt der vorliegenden Erfindung werden Verfahren zur Bewertung der Wirkungen von Verbindungen vorgeschlagen, bei denen eine Verbindung mit einem Cytohesin-PH-Peptid in Kontakt gebracht wird und die Wirkungen der Verbindungen auf die Aktivität von Cytohesin-PH bestimmt werden. So könnte eine Ausführungsform des Tests Cytohesin-PH oder die Prüfsubstanz umfassen, wobei eine dieser Verbindungen an eine unlösliche Matrix, wie zum Beispiel SEPHAROSE®, gebunden ist. Die andere Verbindung wird (radioaktiv, enzymatisch o.ä.) markiert, und ein Direktbindungsversuch wird durchgeführt. Mit diesem Test ist es möglich, Verbindungen zu identifizieren, die an Cytohesin-PH binden und es so möglicherweise blockieren oder hemmen. Verbindungen, die an Cytohesin-PH binden, sollten dann mit den nachstehend beschriebenen Zelltests geprüft werden.

Es werden auch Verfahren zur Bewertung der Wirkungen von Verbindungen mit Cytohesin-PH vorgeschlagen. Diese Verfahren umfassen Zelltests. Ein Zelltest könnte die folgenden Schritte umfassen: Kultivierung der Zellen einer Testgruppe und einer Kontrollgruppe, die die Fähigkeit besitzen, an einem Substrat (wie z.B. eine mit ICAM-1-Rg o.ä. beschichtete Kulturschale) zu haften, wobei die Testgruppe in Gegenwart einer Testverbindung und die Kontrollgruppe in Abwesenheit einer Testverbindung gezüchtet wird; Einleiten der Expression von Cytohesin-PH in den Testzellen und in den Kontrollzellen; und Vergleichen des Ausmaßes des Haftungsverlusts in der Testgruppe und in der Kontrollgruppe. In einem geeigneten Test würden die Zellen der Kontrollgruppe die Haftfähigkeit verlieren. Wenn die Zellen der Testgruppe die Haftfähigkeit in einem geringeren Ausmaß verlieren, interferiert oder blockiert die Prüfsubstanz die anti-adhäsiven Eigenschaften von Cytohesin-PH.

Ein weiterer Gegenstand der Erfindung ist ein Cytohesin-2- Peptid mit der Aminosäuresequenz gemäß Figur 1B.

Ferner ist Gegenstand der Erfindung die Verwendung eines Cytohesin-2-Peptids mit der von der cts 18.1-cDNA kodierten Aminosäuresequenz zur Regulation der T-Lymphozyten-Aktivierung.

Außerdem ist Gegenstand der Erfindung eine DNA, kodierend für ein Cytohesin-2 Peptid oder Teile davon.

Ferner ist Gegenstand der Erfindung eine DNA, dadurch gekennzeichnet, daß deren Sequenz in bezug auf die Sequenz der DNA wie im vorigen Absatz erwähnt, entsprechend dem Wesen des genetischen Codes degeneriert ist.

Ein weiterer Gegenstand der Erfindung ist die Verwendung einer DNA, kodierend für ein Cytohesin-2 Peptid zur Expression desselben.

Ferner ist Gegenstand der Erfindung die Verwendung eines Cytohesin-2 Peptids gemäß Figur 1B zur Beeinflussung von Entzündungen, zur Verbesserung der Wundheilung, zur Suppression des Immunsystems, insbesondere bei Organtransplantationen, zur Verhinderung der Metastasierung bei hämatopoietischen Tumoren und zur Behandlung von Arteriosklerose.

Ein weiterer Gegenstand der Erfindung ist ein Arzneimittel, enthaltend ein Cytohesin-2 Peptid und einen physiologisch annehmbaren Träger sowie gegebenenfalls geeignete Zusatz- und Hilfsstoffe.

Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten, organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.
Bevorzugt sind orale Verabreichung und Injektionen. Für die Injektion werden die Cytohesin-PH-Peptide in einer flüssigen Lösung, vorzugsweise in einem physiologisch annehmbaren Puffer,wie z.B. Hank's Lösung oder Ringer's Lösung, formuliert. Die Cytohesin-PH-Peptide können aber auch in fester Form formuliert werden und vor dem Gebrauch gelöst oder suspendiert werden.

Die für die systematische Verabreichung bevorzugten Dosierungen betragen ca. 0,01 mg/kg bis ca. 50 mg/kg Körpergewicht und Tag.

Ein weiterer Gegenstand der Erfindung ist ein Verfahren zur Herstellung eines oben beschriebenen Cytohesin-PH-Peptids, dadurch gekennzeichnet, daß
a) eine Wirtszelle gemäß Anspruch 7 kultiviert wird und
b) das Cytohesin-PH-Peptid isoliert wird.

Die Erfindung wird nun anhand der Figuren und Beispiele näher erläutert, ohne darauf beschränkt zu sein. Die beispielhaft erläuterten Experimentalschritte werden im folgenden aufgelistet:
1) Herstellung des CD18 cyt Köderkonstruktes
2) Herstellung der Hefe-Expressionsbank
3) Screening mit dem Two-Hybrid-System
4) Test der Bindungsspezifität in Hefe
5) Herstellung der Fusionskonstrukte zum Test der Funktion von Cytohesin-1 in vivo
6) Funktionsassay von Cytohesin-1 und der Subdomänen
7) Herstellung des ICAM-Rg Fusionsproteins
8) Cytohesin-PH-Domänen-spezifische funktionelle Inhibierung von β2-Integrinen

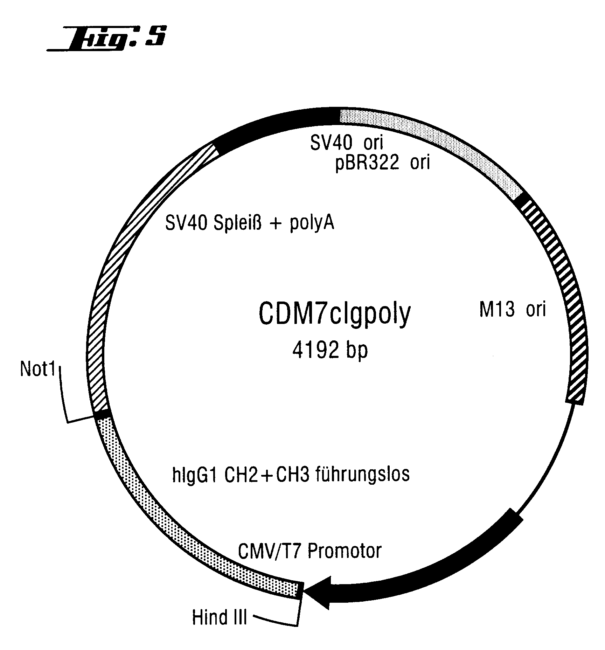

Die Figuren und Tabellen sind wie folgt beschrieben;

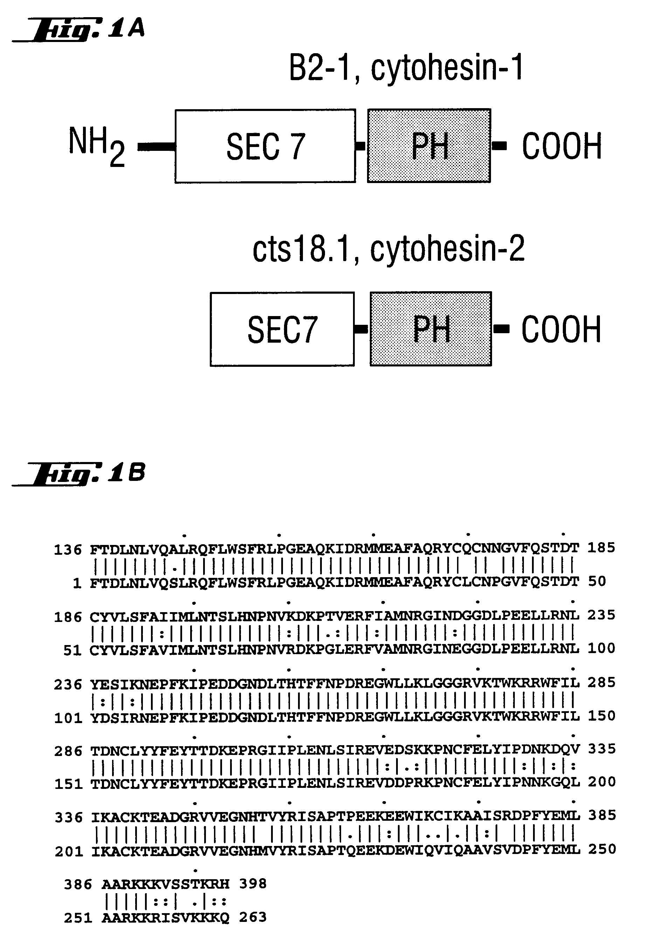

- Fig. 1: Molekulare Klonierung von cts 18.1. Eine Jurkat cDNA-Bibliothek enthaltend 4·10⁶ Klone wurde unter Verwendung des Hefe-Expressionsvektors pJG4-5 hergestellt. Mit einem Aliquot der Bibliothek wurde eine Hefe transformiert, die bereits vorher mit einem LexA-CD18 Fusionsprotein-Expressionsplasmid transformiert worden war. Die 8·10⁵ entstandenen Primärkolonien wurden auf eine Interaktion mit der cytoplasmatischen Domäne von CD18 getestet. Der Klon cts 18.1 und die B2-1 cDNA sind schematisch unter (A) gezeigt, der entsprechende Aminosäurevergleich ist unter (B) gezeigt. Die obere Sequenz ist die B2-1 Aminosäuresequenz beginnend bei AS 136, die untere Sequenz ist die Aminosäuresequenz des cts 18.1 Polypeptids.

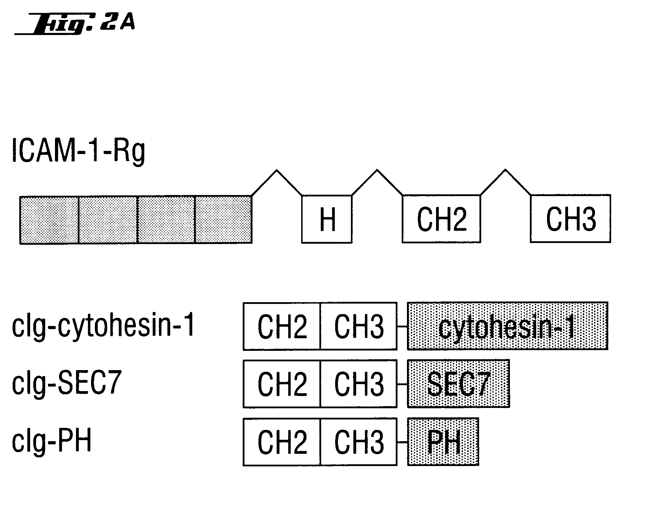

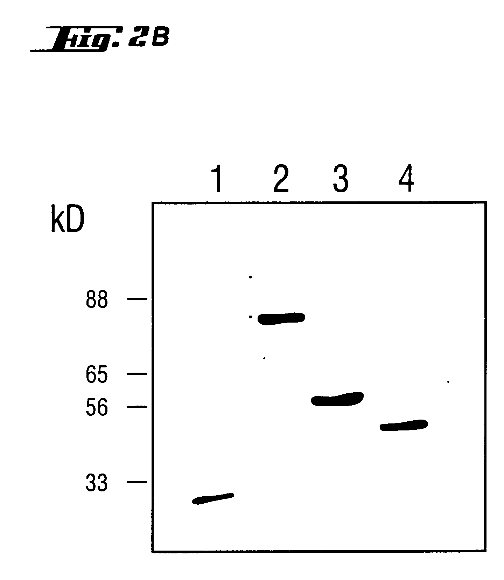

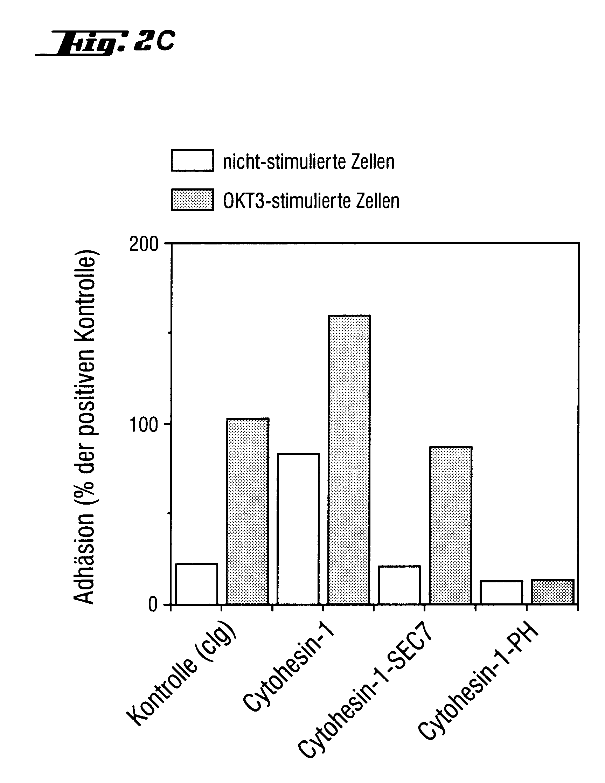

- Fig. 2:: Einfluß von Volle-Länge-Cytohesin-1, PH- und SEC7-Domäne Fusionsproteinen auf die Bindung von J32-Zellen an ICAM-1-Rg (A). Schematische Darstellung der Konstrukte, die im Experiment benutzt wurden. (B) Expression von Cytohesin-1 Fusionsprotein in J32-Zellen. cDNA-Segmente, kodierend für Volle-Länge-Cytohesin-1, SEC7- und PH-Domänen-Sequenzen wurden in einen Vaccinia Virus Expressionsvektor kloniert, der eine Expressions-Cassette für die intrazelluläre Ig-Fusionsprotein-Expression enthielt. Die Konstrukte wurden mit Wildtyp Vaccinia-Virus (WR) in CV-1 Zellen rekombiniert. Rekombinante Plaques wurden isoliert und hochtitrige Virus-Stocklösungen erzeugt. 5·10⁶ Jurkat J32-Zellen wurden damit infiziert und in RPMI-Medium (10 % fötales Rinderserum, Moore et al., JAMA 199: 519-24, 1967) für 4 Stunden inkubiert. Die Zellen wurden dann in 150 mM NaCl, 100 mM Tris Cl pH 7,5, 1 % Triton-X-100, 1 mM PMSF lysiert und die Fusionsproteine wurden durch Protein-A-Sepharose®-Kügelchen gebunden. Aliquote der eluierten Proteine wurden durch Polyacrylamidelektrophorese aufgetrennt und auf Nitrozellulose geblottet. Die Fusionsproteine wurden durch Inkubation der Filter mit Protein-A-Peroxidase konjugiert und anschließende Behandlung mit einem Chemilumineszenz-Substrat von einem entsprechenden Assay (Chemilumineszenzkit von Amersham: ECL-Kit, RPN-2106) sichtbar gemacht. Legende: (1) cIg-Kontrolle (2) cIg-Cytohesin-1 (3) cIg-sec7 (4) cIg-PH. (C) Adhäsions-Assay. ICAM-1-Rg Fusionsprotein wurde in COS-Zellen exprimiert und aus dem Kulturüberstand mit Protein-A-Sepharose® isoliert, dann eluiert und in PBS (Phosphate Buffered Saline) resuspendiert. Das ICAM-1-Rg wurde dann zum Beschichten("Coaten") von Falcon® 1008 Plastikschalen verwendet wie beschrieben (D. Rawlings et al., Science 261: 358-361, 1993). Jurkat 32-Zellen wurden dann wie unter (B) beschrieben mit rekombinantem Vaccinia Virus infiziert. Aliquots dieser Zellen wurden dann mit dem Fachmann bekannten Methoden permeabilisiert und mit einem anti-IgG-FITC-Konjugat (Ziege-anti-Mensch-Fluorescein-Isothiocyanat; Hersteller: Jackson Labs; Vertrieb, DIANOVA, Hamburg, Code: 109-095-088) gefärbt. Die Expression wurde durch eine cytometrische Fließanalyse (Gerät: Coulter Epics XL) beobachtet; normalerweise waren mehr als 30 % der Zellen positiv. 2·10⁶-Zellen wurden in RPMI-Medium für 5 min bei 25°C mit oder ohne Zugabe von 3 µg/ml OKT3 Antikörper inkubiert. Ein Vergleichstest mit einem Kontrollantikörper (mIgG2b) zeigte kein Effekt (hier nicht gezeigt). Nachfolgend wurden die so behandelten Zellen auf ICAM-1-Rg beschichtete Plastikschalen (25°C, 5 min) aufgebracht und der gebundene Teil der Zellen mittels eines Mikroskops bestimmt. Das repräsentative Ergebnis eines Experiments von insgesamt 8 unabhängigen Experimenten ist dargestellt.

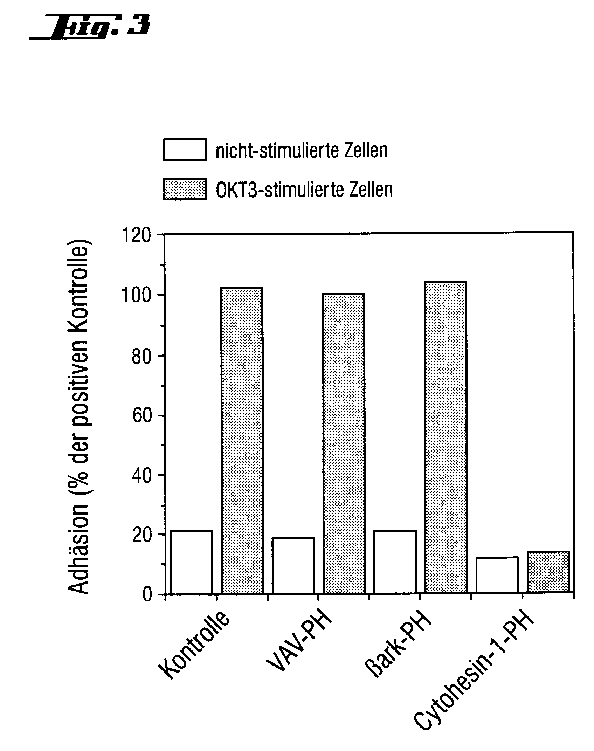

- Fig. 3:: Spezifität der Cytohesin-1-PH Domäne für die Bindung von J32-Zellen an ICAM-1-Rg. cDNA-Fragmente, die für die PH-Domänen von βark-(Benovic et al., FEBS Lett. 283; 122-126, 1991; Nukleotide 1763-2075) und VAV-Protein (Katzav et al., EMBO J. 8: 2283-2290, 1989; Nukleotide 1152-1484) kodieren, wurden in das beschriebene Vaccinia Virus Expressionssystem eingebracht. Der Einfluß der Expression der entsprechenden PH-Domänen auf die Bindung von J32-Zellen an ICAM-1-Rg wurde getestet wie bei Fig. 2 beschrieben. Ein Experiment von drei unabhängig voneinander gemachten Experimenten ist gezeigt.

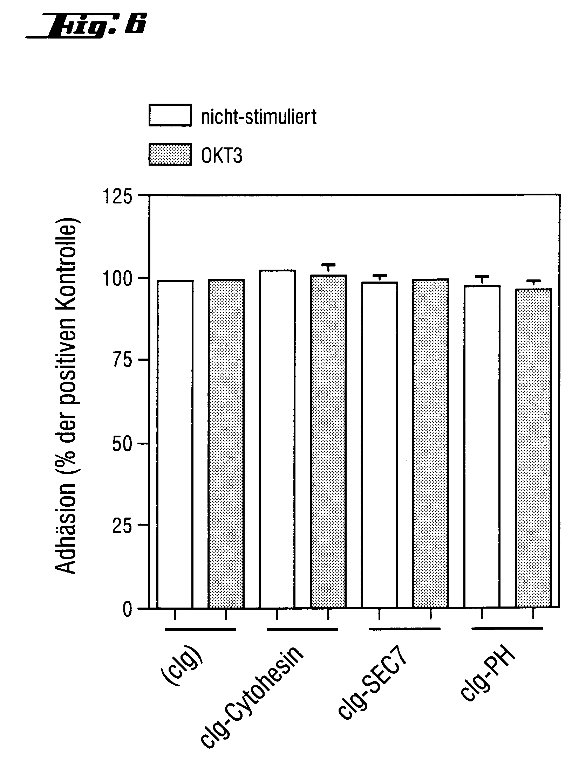

- Fig. 5:: Karte des Vektors CDMcIg poly
- Fig. 6:: Bindung von J32-Zellen an VCAM-1. Es gilt in Prinzip das in der Legende zu Figur 3 angegebene, jedoch hier bezogen auf die Bindung von J32-Zellen an VCAM-1.
- Tab.1:: Spezifität der Interaktion zwischen CD18 cyt und cts 18.1. Der Klon cts 18.1 wurde in Hefe-Zellen transformiert, die schon ein Expressionskonstrukt, kodierend die β₂-cytoplasmatische Domäne, oder mehrere andere transmembrane cytoplasmatische Domänen (-cyt), einschließlich der β₁-Integrin-cytoplasmatischen Domäne (CD29 cyt) sowie einiger Kontrollproteine, enthielten. Die Wechselwirkung wurde nach dem gleichen Verfahren wie in der Figurenlegende zu Figur 4 beschrieben, bestimmt.
- Tab. 2:: Cytohesin-1-cDNA. Dargestellt sind beide DNA-Stränge mit entsprechender Numerierung sowie die Aminosäuresequenz des von der cDNA abzuleitende Cytohesin-1-Proteins mit entsprechender Aminosäure-Numerierung. Das Stopkodon ist durch ein "Sternchen" dargestellt.

### Beispiel 1 Herstellung des CD18 cyt Köderkonstruktes

### Primer mit den Sequenzen

| | | |
|---|---|---|
| cgc ggg acg cgt gct ctg atc cac ctg agc | CD18 cyt for | (SEQ ID NO.: 1) |
| cgc ggg gcg gcc gct tta act ctc agc aaa ctt ggg | CD18 cyt rev | (SEQ ID NO.: 2) |

wurden benutzt, um die cytoplasmatische Domäne von CD18 aus der Volle-Länge Version eines cDNA-Klons von CD18 (Brian Seed, unpubliziert, entspricht der Sequenz von CD18 in Kishimoto et al., Cell 48, 681-690, 1987) durch PCR zu amplifizieren. Die PCR-DNA wurde mit den Restriktionsenzymen MluI und NotI verdaut und das Produkt in den nach gängigen molekularbiologischen Methoden vorbereiteten Vektor pLex202 (J. Gyuris, E. Golemis, H. Chertkov, R. Brent, Cell 75, 791-803, 1993) inseriert. Die Sequenzidentät wurde durch doppelsträngige Sequenzierung verifiziert. Das erhaltene Konstrukt wurde lex 202-cd18 genannt.

### Beispiel 2 Herstellung der Hefe-Expressionsbank

Poly-A-RNA wurde gemäß Ausubel et al. (Current Protocols in Molecular Biology, Wiley Interscience, New York, 1987) aus Jurkat E6 Zellen (ATCC TIB-152) gereinigt und in vitro revers transkribiert (1). Die doppelsträngige cDNA wurde mit EcoRI Adaptern versehen, mit XhoI verdaut und in den EcoRI-XhoI verdauten Vektor pJG4-5 ligiert. Der Bakterienstamm MC1061 wurde anschließend mit dieser Ligationsmixtur transformiert. Sowohl der Vektor pJG4-5 als auch der Bakterienstamm MC1061 sind aus der schon zitierten Arbeit von Gyuris et al. (1993) bekannt. Die initiale Amplifikation der Bibliothek erbrachte 4 x 10⁶ rekombinante Klone. Die Bakterienzellen wurden anschließend lysiert und die doppelsträngige Plasmid-DNA als "library-stock" präpariert (Ausubel et al., 1987).

### Beispiel 3 Screening mit dem Two-Hybrid-System

Das lex202-cd18 Konstrukt aus Beispiel 1 wurde durch LiCl-Transformation (Ausubel et al., 1987) in den Hefestamm EG048JK103 (Gyuris et al., 1993) transformiert. Der hierbei entstehende Stamm EG048JK103CD18 wurde zur Bibliotheken-Transformation mit Hilfe der LiCl-Methode kompetent gemacht. In diese kompetente Zellen wurden 50 µg der Bibliothek transformiert, was schließlich ca. 900 000 unabhängige rekombinante Hefeklone auf URA-HIS-TRP-Medien erbrachte. Aliquots dieser Hefebibliothek (ca. 20 x 10⁶ Zellen) wurden auf URA(-)HIS(-)TRP(-)LEU(-)XGAL Indikatorplatten ausplattiert und positive Klone (blau) wurden selektiert. Anschließend wurde Plasmid-DNA aus den Hefezellen präpariert (Ausubel et al., 1987) und doppelsträngig sequenziert. Dabei wurde die cDNA pJG4-5cts18.1, kurz cts18.1. genannt, erhalten, die auf Proteinebene 97 % homolog zu hsec7hom (Accesion #:Genbank M85169) ist. hsec7hom wird im folgenden cytohesin-1, cts18.1 wird cytohesin-2 genannt (vgl. Figur 1). Die cDNA zu Cytohesin-1- wird als B2-1 cDNA bezeichnet (Liu und Pohajdak, 1992).

### Beispiel 4 Test der Bindungsspezifität in Hefe

pJG4-5cts18.1 wurde in Hefe EG048JK103 transformiert (s. Beispiel 3) und der daraus resultierende Stamm EG048JK103cts18.1 zum Test der Bindungsspezifität kompetent gemacht (Ausubel et al., 1987). Diese kompetenten Hefezellen wurden mit verschiedenen lex202-Konstrukten transformiert, die in analoger Weise zum CD18-Köderkonstrukt hergestellt worden waren (lex202-CD 29b, -CD2, -CD4, -CD8, IdIreceptor, -HIV-rev, -HIV-tat, -fyn, -syk, -ZAP-70). Die Hefezellen wurden auf URA(-), HIS(-), TRP(-)Medien ausplattiert und positive Klone auf URA(-)HIS(-)TRP(-)LEU(-)XGAL Indikatormedien getestet. Als Testkriterium wurde die Blaufärbung der Hefezellen herangezogen. Die Ergebnisse sind in der Tabelle 1 zusammengefaßt.

### Beispiel 5 Herstellung der Fusionskonstrukte zum Test der Funktion von cytohesin-1 in vivo

### Cytohesin-1 wurde mit Hilfe der Primer

| | | |
|---|---|---|
| cgc ggg acg cgt atg gag gag gac gac agc tac gtt ccc | hsec7hom mlu for | (SEQ ID NO.: 3) |
| cgc ggg gcg gcc gct tta gtg tcg ctt cgt gga gga gac ctt | hsec7hom not rev | (SEQ ID NO.: 4) |

aus einer natürlichen Killerzell-Bibliothek amplifiziert, über die Restriktionsschnittstellen MluI und NotI in den Vektor pCDM7cIgpoly (Abbildung 5) kloniert und sequenziert. Die Sequenzen, welche die PH und SEC7 Subdomänen kodieren wurden in analoger Weise aus der cytohesin-1 Sequenz PCR-amplifiziert und in pCDM7cIgpoly inseriert. Primer:

| | | |
|---|---|---|
| gcg ggg acg cgt acc atg gct aat gaa att gaa aac ctg | sec7 mlu nco for | (SEQ ID NO.: 5) |
| gcg ggg gcg gcc gct tta gaa agt gtg agt gag gtc att ccc | sec7 not rev | (SEQ ID NO.: 6) |
| cgc ggg acg cgt acc atg ggt ttc aat cca gac cga gaa ggc tgg | ph mlu ncofor | (SEQ ID NO.: 7) |
| cgc ggg gcg gcc gct tta gtg tcg ctt cgt gga gga gac ctt | hsec7hom not rev | (SEQ ID NO.: 8) |

Das Konstrukt zur Expression von ICAM-1 Rg wurde in analoger Weise hergestellt, nur dient hier die Expressionskassette zur Expression eines sezernierten Immunglobulinfusionskonstruktes. Herstellung der sezernierten Ig-Kassette beschrieben in G. Walz,A.Aruffo, W. Kolanus, M. Bevilacqua, B.Seed, Science 250: 1132-35 (1990).

### Beispiel 6 Funktionsassay von Cytohesin-1 und der Subdomänen

Die cDNA Segmente, die für cytohesin-1 und die jeweiligen Subdomänen kodieren, wurden zusammen mit der cIg-Kassette in den Vaccinia-Expressionsvektor ptkg (W. Kolanus, C. Romeo, B. Seed, Cell 74: 171-83, 1993), inseriert.
Diese Vektoren wurden in CV-1-(ATCC70-CCL) Zellen, die mit Wildtyp Vaccinia-Virus (WR) infiziert worden waren, transfiziert. Rekombinante Viren wurden durch gpt-Selektion erhalten und auf CV-1 Zellen amplifiziert. Für die Experimente wurden pro Wert 5 x 10⁶ Jurkat J32 (W. Tadmorei, J.A. Kant, M. Kamoun, J. Immunol. 136(4), 1155-60, 1986) Zellen mit je 100 µl Virusstock infiziert, dessen Titer mindestens 5 x 10⁷ pfu/ml betragen mußte, und anschließend für vier Stunden in RPMI/10%FCS inkubiert. Die Zellen wurden anschließend abzentrifugiert und für 5 min in Anwesenheit oder Abwesenheit einer Konzentration von 3 mg/ml OKT3 Antikörper (gereinigt aus Hybridomüberständen, Herkunft des Hybridoms: ATCC CRL-8001) bei Raumtemperatur inkubiert. Die Suspension wurde anschließend auf mit ICAM-1-Rg beschichtete Zellkulturschalen (Falcon® 1008) pipettiert und weiter 10 min inkubiert. Nach dreimaligem Waschen mit Medium wurden die gebundenen Zellen fixiert und unter dem Mikroskop ausgezählt. Die Ergebnisse sind in Figur 2 dargestellt.

### Beispiel 7 Herstellung des ICAM-Rg Fusionsproteins

ICAM-1-Rg cDNA wurde durch DEAE-Dextran-Transfektion für 10 Tage in cosM6 Zellen exprimiert (Walz et al. 1990, cosM6: Subklon von cos7; Herkunft cos7: ATCC CRL-1651; cosM6 selektiert für gute Transfizierbarkeit). Anschließend wurden die Überstände geerntet und mit Hilfe von Protein-A-Sepharose® (Sigma) aufgereinigt. Das gebundene Protein wurde mit Hilfe von 4M Imidazol-Lösung eluiert und nach Dialyse gegen PBS-Puffer in einer Konzentration von ca. 0,2µg/ml aufbewahrt.
Falcon® 1008 Schalen wurden mit einer Schaf-anti-Mensch Antikörperpräparation beschichtet an die dann im zweiten Schritt ICAM-1-Rg gebunden wurde (Walz et al., 1990). Diese Schalen dienten zur Bestimmung der Cytohesin-Funktion, wie in Beispiel 6 beschrieben.

### Beispiel 8 Cytohesin-PH-Domänen-spezifische funktionelle Inhibierung von β2-Integrinen

Die Cytohesin-PH-Domäne inhibiert spezifisch die Funktion von beta-2 Integrinen. Die Bindung von beta-1 Integrin an dessen Liganden VCAM-1 (Osborn et al., Cell 59 (1989) 1203-1211) wird von Cytohesin-PH nicht beeinflußt. Das für den Adhäsionsassay verwendete VCAM-1-Rg Fusionsprotein ist nach dem gleichen Muster konstruiert wie ICAM-1-Rg. Die Beschreibung des Assays ist identisch wie bei Beispiel 7. Die Expression der Cytohesin-Konstrukte erfolgt wie in Beispiel 6 beschrieben. Die Abbildung 6 zeigt, daß J32 Zellen über beta-1 Integrine konstitutiv an VCAM-1 binden. Cytohesin-Fusionsproteine zeigen dabei keinerlei Effekt.

### Beispiel 9 Kartierung der Cytohesin-Interaktions-Domänen.

Die Cytohesin-Interaktions-Domäne in bezug auf CD 18 Cyt wurde in Hefe kartiert. Die Sequenz cts 18.1 und 2 PCR-Fragmente, die die SEC7- und PH-Domänen von Cytohesin-1 enthielten, wurden in den Hefe-Expressionsvektor PJG4-5 kloniert. Die Konstruktionen wurden in Hefezellen eingeführt, die auf einem Medium ausgestrichen wurden, das X-GAL, einen Farbindikator, enthält. Blaufärbung ist ein Zeichen für eine Wechselwirkung zwischen den Fusionsproteinen.

Es hat sich gezeigt, daß eine reproduzierbare Interaktion der SEC7-Domäne mit CD18 bestand, während eine Interaktion von CD18 mit der PH-Domäne nicht nachgewiesen werden konnte. Die Ergebnisse sind in der nachstehenden Tabelle A zusammengefaßt.

**Tabelle A**

| Konstruktionen | CD 18 cyt |
|---|---|
| (a) Vektor PJG 4-5 allein (Negativ-Kontrolle) | weiß |
| (b) PJG 4-5 (cts 18,1) | hellblau |
| (c) PJG 4-5 (Cytohesin-1 PH-Domäne) | weiß |
| (d) PJG 4-5 (Cytohesin-1 SEC 7-Domäne) | blau |

Da die PH-Domäne sehr heftig mit der β2-Integrin-Funktion interferiert, aber, wie aus Tabelle 1 zu ersehen ist, nicht an CD18 in der Hefe bindet, scheint diese Domäne Elemente der "inside-out"-Signalwege der Integrins zu koppeln.

Anhang

Tabelle 1

| pJG4-5-Derivate
plex202-Derivate | cytohesin1-SEC7

lex-CD18cyt | blau
lex-CD29cyt | weiß
lex-CD2cyt | weiß
lex-CD4cyt | weiß
lex-CD8cyt | weiß
lex-ldlrcyt | weiß
lex-rev | weiß
lex-tat | weiß
lex-fyn | weiß
lex-syk | weiß
lex-ZAP70 | weiß

Tabelle 2 (SEQ NO.: 9 + 10)

GCGAGCGGGGGCGCGGGTGGCGCGGCGGGACGCGAGCGGCGAGCCGGAGCGCGAGCCCGC
1 ---------+---------+---------+---------+---------+---------+ 60
CGCTCGCCCCCGCGCCCACCGCGCCGCCCTGCGCTCGCCGCTCGGCCTCGCGCTCGGGCG

TCCCGCACCATGGAGGAGGACGACAGCTACGTTCCCAGTGACCTGACAGCAGAGGAGCGT
61 ---------+---------+---------+---------+---------+---------+ 120
AGGGCGTGGTACCTCCTCCTGCTGTCGATGCAAGGGTCACTGGACTGTCGTCTCCTCGCA

1 M E E D D S Y V P S D L T A E E R 17

CAAGAACTGGAGAACATCCGACGGAGAAAACAGGAGCTGCTGGCTGACATTCAGAGGCTG
121 ---------+---------+---------+---------+---------+---------+ 180
GTTCTTGACCTCTTGTAGGCTGCCTCTTTTGTCCTCGACGACCGACTGTAAGTCTCCGAC

18 Q E L E N I R R R K Q E L L A D I Q R L 37

AAGGATGAGATAGCAGAAGTAGCTAATGAAATTGAAAACCTGGGATCCACAGAGGAAAGG
181 ---------+---------+---------+---------+---------+---------+ 240
TTCCTACTCTATCGTCTTCATCGATTACTTTAACTTTTGGACCCTAGGTGTCTCCTTTCC

38 K D E I A E V A N E I E N L G S T E E R 57

AAAAACATGCAGAGGAACAAACAGGTAGCCATGGGCAGGAAAAAATTTAATATGGACCCT
241 ---------+---------+---------+---------+---------+---------+ 300
TTTTTGTACGTCTCCTTGTTTGTCCATCGGTACCCGTCCTTTTTTAAATTATACCTGGGA

58 K N M Q R N K Q V A M G R K K F N M D P 77

AAAAAGGGGATCCAGTTCTTAATAGAGAACGACCTCCTGAAGAACACTTGTGAAGACATT
301 ---------+---------+---------+---------+---------+---------+ 360
TTTTTCCCCTAGGTCAAGAATTATCTCTTGCTGGAGGACTTCTTGTGAACACTTCTGTAA

78 K K G I Q F L I E N D L L K N T C E D I 97

GCCCAGTTCTTATATAAAGGCGAAGGGCTCAACAAGACAGCCATCGGCGACTACCTAGGG
361 ---------+---------+---------+---------+---------+---------+ 420
CGGGTCAAGAATATATTTCCGCTTCCCGAGTTGTTCTGTCGGTAGCCGCTGATGGATCCC

98 A Q F L Y K G E G L N K T A I G D Y L G 117

GAGAGAGATGAGTTTAATATCCAGGTTCTTCATGCATTTGTGGAGCTGCATGAGTTCACT
421 ---------+---------+---------+---------+---------+---------+ 480
CTCTCTCTACTCAAATTATAGGTCCAAGAAGTACGTAAACACCTCGACGTACTCAAGTGA

118 E R D E F N I Q V L H A F V E L H E F T 137

GATCTTAATCTCGTCCAGGCACTACGGCAGTTCCTGTGGAGCTTCCGGCTACCCGGAGAG
481 ---------+---------+---------+---------+---------+---------+ 540
CTAGAATTAGAGCAGGTCCGTGATGCCGTCAAGGACACCTCGAAGGCCGATGGGCCTCTC

138 D L N L V Q A L R Q F L W S F R L P G E 157

GCCCAGAAGATCGACCGGATGATGGAGGCGTTTGCCCAGCGATATTGTCAGTGCAATAAT
541 ---------+---------+---------+---------+---------+---------+ 600
CGGGTCTTCTAGCTGGCCTACTACCTCCGCAAACGGGTCGCTATAACAGTCACGTTATTA

158 A Q K I D R M M E A F A Q R Y C Q C N N 177

GGCGTGTTCCAGTCCACGGATACTTGTTACGTCCTCTCCTTTGCCATCATCATGTTGAAC
601 ---------+---------+---------+---------+---------+---------+ 660
CCGCACAAGGTCAGGTGCCTATGAACAATGCAGGAGAGGAAACGGTAGTAGTACAACTTG

178 G V F Q S T D T C Y V L S F A I I M L N 197

ACCAGTCTGCACAACCCCAATGTCAAAGATAAGCCCACTGTGGAGAGGTTCATTGCCATG
661 ---------+---------+---------+---------+---------+---------+ 720
TGGTCAGACGTGTTGGGGTTACAGTTTCTATTCGGGTGACACCTCTCCAAGTAACGGTAC

198 T S L H N P N V K D K P T V E R F I A M 217

AACCGAGGCATCAATGATGGGGGAGACCTGCCGGAGGAGCTCCTCCGGAATCTCTATGAG
721 ---------+---------+---------+---------+---------+---------+ 780
TTGGCTCCGTAGTTACTACCCCCTCTGGACGGCCTCCTCGAGGAGGCCTTAGAGATACTC

218 N R G I N D G G D L P E E L L R N L Y E 237

AGCATAAAAAATGAACCCTTTAAAATCCCAGAAGACGACGGGAATGACCTCACTCACACT
781 ---------+---------+---------+---------+---------+---------+ 840
TCGTATTTTTTACTTGGGAAATTTTAGGGTCTTCTGCTGCCCTTACTGGAGTGAGTGTGA

238 S I K N E P F K I P E D D G N D L T H T 257

TTCTTCAATCCAGACCGAGAAGGCTGGCTATTGAAACTCGGAGGTGGCAGGGTAAAGACT
841 ---------+---------+---------+---------+---------+---------+ 900
AAGAAGTTAGGTCTGGCTCTTCCGACCGATAACTTTGAGCCTCCACCGTCCCATTTCTGA

258 F F N P D R E G W L L K L G G G R V K T 277

TGGAAGAGACGCTGGTTCATTCTGACTGACAACTGCCTTTACTACTTTGAGTATACCACG
901 ---------+---------+---------+---------+---------+---------+ 960
ACCTTCTCTGCGACCAAGTAAGACTGACTGTTGACGGAAATGATGAAACTCATATGGTGC

278 W K R R W F I L T D N C L Y Y F E Y T T 297

GATAAGGAGCCCCGTGGAATCATCCCTTTAGAGAATCTGAGTATCCGGGAAGTGGAGGAC
961 ---------+---------+---------+---------+---------+---------+ 1020
CTATTCCTCGGGGCACCTTAGTAGGGAAATCTCTTAGACTCATAGGCCCTTCACCTCCTG

298 D K E P R G I I P L E N L S I R E V E D 317

TCCAAAAAACCAAACTGCTTTGAGCTTTATATCCCCGACAATAAAGACCAAGTTATCAAG
1021 ---------+---------+---------+---------+---------+---------+ 1080
AGGTTTTTTGGTTTGACGAAACTCGAAATATAGGGGCTGTTATTTCTGGTTCAATAGTTC

318 S K K P N C F E L Y I P D N K D Q V I K 337

GCCTGCAAGACCGAGGCTGACGGGCGGGTGGTGGAGGGGAACCACACTGTTTACCGGATC
1081 ---------+---------+---------+---------+---------+---------+ 1140
CGGACGTTCTGGCTCCGACTGCCCGCCCACCACCTCCCCTTGGTGTGACAAATGGCCTAG

338 A C K T E A D G R V V E G N H T V Y R I 357

TCAGCTCCGACGCCCGAGGAGAAGGAGGAGTGGATTAAGTGCATTAAAGCAGCCATCAGC
1141 ---------+---------+---------+---------+---------+---------+ 1200
AGTCGAGGCTGCGGGCTCCTCTTCCTCCTCACCTAATTCACGTAATTTCGTCGGTAGTCG

358 S A P T P E E K E E W I K C I K A A I S 377

AGGGACCCTTTCTACGAAATGCTCGCAGCACGGAAAAAGAAGGTCTCCTCCACGAAGCGA
1201 ---------+---------+---------+---------+---------+---------+ 1260
TCCCTGGGAAAGATGCTTTACGAGCGTCGTGCCTTTTTCTTCCAGAGGAGGTGCTTCGCT

378 R D P F Y E M L A A R K K K V S S T K R 397

CACTGAGCGTGCAGCCAAGGGCGTTGGTCTGCGGGGGCCTTGGAGCTCCTGCTCTTCTCC
1261 ---------+---------+---------+---------+---------+---------+ 1320
GTGACTCGCACGTCGGTTCCCGCAACCAGACGCCCCCGGAACCTCGAGGACGAGAAGAGG

398 H *

CGCACCTCCATGGATGCACTGCTGCCGAGCAGAGCGTCCTCTGCCAGGCCCCGCCCTGGA
1321 ---------+---------+---------+---------+---------+---------+ 1380
GCGTGGAGGTACCTACGTGACGACGGCTCGTCTCGCAGGAGACGGTCCGGGGCGGGACCT

TTCCTAGAGACTAGCTTCAGCTTTTGCTATTTTTTTAAGTGGGAGAAGGGTGGGCAGTT
1381 ---------+---------+---------+---------+---------+---------+ 1440
AAGGATCTCTGATCGAAGTCGAAAACGATAAAAAAATTCACCCTCTTCCCACCCGTCAA

ATCACTGGGGAAGAGAGGACCGGCCACCTGTCCAGCATGGGCTCCAGAGCCTTCCTCTCT
1441 ---------+---------+---------+---------+---------+---------+ 1500
TAGTGACCCCTTCTCTCCTGGCCGGTGGACAGGTCGTACCCGAGGTCTCGGAAGGAGAGA

CACAGGGCAGAGCTCTTGTCGGCAGGGCAGCCTCCTGGCCAGTTTCTCTGCTCAGTGTTC
1501 ---------+---------+---------+---------+---------+---------+ 1560
GTGTCCCGTCTCGAGAACAGCCGTCCCGTCGGAGGACCGGTCAAAGAGACGAGTCACAAG

TGGTAGCAGAGCTCAGAGCCAACTGTTTACCTCTTGGTTGTCCCCGTGAAGAAGCCTTCA
1561 ---------+---------+---------+---------+---------+---------+ 1620
ACCATCGTCTCGAGTCTCGGTTGACAAATGGAGAACCAACAGGGGCACTTCTTCGGAAGT

AACCCTGCACCATAAATACATGTGTCCATATATTATTATATGTTAAGAGAAAAAGGTGGA
1621 ---------+---------+---------+---------+---------+---------+ 1680
TTGGGACGTGGTATTTATGTACACAGGTATATAATAATATACAATTCTCTTTTTCCACCT

AAGGAAGAGAAGCCACATACTATAAAGATCTATTTTTTTTTTTAAGAGAGAACGTAGGG
1681 ---------+---------+---------+---------+---------+---------+ 1740
TTCCTTCTCTTCGGTGTATGATATTTCTAGATAAAAAAAAAAAATTCTCTCTTGCATCCC

CTGTTCAGGTGCATTCTGCCCTGGCTGCGCTGGGGAGCTTCTCCCTGGAGAAGAGCACCT
1741 ---------+---------+---------+---------+---------+---------+ 1800
GACAAGTCCACGTAAGACGGGACCGACGCGACCCCTCGAAGAGGGACCTCTTCTCGTGGA

GGGGCTGCGGCCAAGGGGCATCAGCCTGGGCCCGCGGCAGGGCCTGGCCTGCCTCTCCTG
1801 ---------+---------+---------+---------+---------+---------+ 1860
CCCCGACGCCGGTTCCCCGTAGTCGGACCCGGGCGCCGTCCCGGACCGGACGGAGAGGAC

TGCTGTGGGAGCTCGCTGCCTGGTGCTTGTCTTGGCGAGATGGACAGGTGAGGTCGAGGA
1861 ---------+---------+---------+---------+---------+---------+ 1920
ACGACACCCTCGAGCGACGGACCACGAACAGAACCGCTCTACCTGTCCACTCCAGCTCCT

CGCAGAGGGCAGAGGCCCAGTGGAGCCTCAGACGGCACAGTCAGAGTCGGGGGCCTGCCT
1921 ---------+---------+---------+---------+---------+---------+ 1980
GCGTCTCCCGTCTCCGGGTCACCTCGGAGTCTGCCGTGTCAGTCTCAGCCCCCGGACGGA

GGCCGGGGTCGCAGTCGGCAGCAGCGTGCAGTCCGGCATCTCCCGCGGATGCTTTTCCAT
1981 ---------+---------+---------+---------+---------+---------+ 2040
CCGGCCCCAGCGTCAGCCGTCGTCGCACGTCAGGCCGTAGAGGGCGCCTACGAAAAGGTA

CCCAAGTGCCTGCGGAGCCCGAGGAGAGGAGAGAGCTGACTGGACGCTTACGTTATTTTC
2041 ---------+---------+---------+---------+---------+---------+ 2100
GGGTTCACGGACGCCTCGGGCTCCTCTCCTCTCTCGACTGACCTGCGAATGCAATAAAAG

CTCCTTCAGAATCCAAGTTCTTGTTGGGCTTTAAAGTAGAAAGTCAGCATTTTCCTTGAG
2101 ---------+---------+---------+---------+---------+---------+ 2160
GAGGAAGTCTTAGGTTCAAGAACAACCCGAAATTTCATCTTTCAGTCGTAAAAGGAACTC

CTAAATACCTAATAACCAAAACTGTGAGGAAGGTTATCGGGACAGAGGTTCCGGATAACC
2161 ---------+---------+---------+---------+---------+---------+ 2220
GATTTATGGATTATTGGTTTTGACACTCCTTCCAATAGCCCTGTCTCCAAGGCCTATTGG

TGTTTCATTTTGGGTTTTCTTCCTCTTCCCCAGACTCCAGTCCTCGTTCTAGAGGAAGGA
2221 ---------+---------+---------+---------+---------+---------+ 2280
ACAAAGTAAAACCCAAAAGAAGGAGAAGGGGTCTGAGGTCAGGAGCAAGATCTCCTTCCT

GTAGGACTTCCCCGATCCCCGTAGCTTCAGCTTTTTCTGCCTCAAAACCAGCCCTAACTG
2281 ---------+---------+---------+---------+---------+---------+ 2340
CATCCTGAAGGGGCTAGGGGCATCGAAGTCGAAAAAGACGGAGTTTTGGTCGGGATTGAC

GACTACTCTGGATGCATTTTGTGGTGGGCCCCCTAGAGGGAAGATGGGCCTTTATCTGCT
2341 ---------+---------+---------+---------+---------+---------+ 2400
CTGATGAGACCTACGTAAAACACCACCCGGGGGATCTCCCTTCTACCCGGAAATAGACGA

CCGTGGGGTGCACTGGAGTGAGGGGGGTGGCCGGGCTGCCTCTCGCATCTCTGTCTTCCC
2401 ---------+---------+---------+---------+---------+---------+ 2460
GGCACCCCACGTGACCTCACTCCCCCCACCGGCCCGACGGAGAGCGTAGAGACAGAAGGG

CTGCAGGCGCTGTGTGAGCTGGCCCTGCCCCTCCTCATTACAGTATGAAGGGAGCCGTGA
2461 ---------+---------+---------+---------+---------+---------+ 2520
GACGTCCGCGACACACTCGACCGGGACGGGGAGGAGTAATGTCATACTTCCCTCGGCACT

CACGCAGCATTTTCCTGCCGTTCTCTCAGGGACTCTCAGGGCAGCTCCTGCCACTCCGCC
2521 ---------+---------+---------+---------+---------+---------+ 2580
GTGCGTCGTAAAAGGACGGCAAGAGAGTCCCTGAGAGTCCCGTCGAGGACGGTGAGGCGG

AGGGCCAGCATGCCAGTCCAGGCAGAGCAGGTGGCTGGCTGTCTGGCCGTCTCGCCCCGC
2581 ---------+---------+---------+---------+---------+---------+ 2640
TCCCGGTCGTACGGTCAGGTCCGTCTCGTCCACCGACCGACAGACCGGCAGAGCGGGGCG

CCCTCCACAGGACCCTGGACCAGGGCGGTGCAGGGCGCAGCCCCGAGGAGGCAGGTGGAG
2641 ---------+---------+---------+---------+---------+---------+ 2700
GGGAGGTGTCCTGGGACCTGGTCCCGCCACGTCCCGCGTCGGGGCTCCTCCGTCCACCTC

GAGCTGCGGGTTTTCACAGGGCCGCGTCGCCACGGCTCCTCTGATCCTTTAGGGTTGGCG
2701 ---------+---------+---------+---------+---------+---------+ 2760
CTCGACGCCCAAAAGTGTCCCGGCGCAGCGGTGCCGAGGAGACTAGGAAATCCCAACCGC

AGCATCTCTGGAAATAGCTTTTGCAGAGGAGTGGTGGGAGGAATAGAGGGGGACAGTCTG
2761 ---------+---------+---------+---------+---------+---------+ 2820
TCGTAGAGACCTTTATCGAAAACGTCTCCTCACCACCCTCCTTATCTCCCCCTGTCAGAC

TCACCTCCCTCCCCGCCACTTTGTGTAGATCCTACCTGGAGGGAATGGCTTTAGGCACTT
2821 ---------+---------+---------+---------+---------+---------+ 2880
AGTGGAGGGAGGGGCGGTGAAACACATCTAGGATGGACCTCCCTTACCGAAATCCGTGAA

TTGTGCCAGAGCTTGTGAGGGTGACAGAAGAGGGTCCAGGCTGGAAACCTGAACTTTCTG
2881 ---------+---------+---------+---------+---------+---------+ 2940
AACACGGTCTCGAACACTCCCACTGTCTTCTCCCAGGTCCGACCTTTGGACTTGAAAGAC

GGTGGGAGAACCAGGTGGTGCCTGCCGAGGTCTGGGCGTGTTTGGGCCGGTGCTGGAGCC
2941 ---------+---------+---------+---------+---------+---------+ 3000
CCACCCTCTTGGTCCACCACGGACGGCTCCAGACCCGCACAAACCCGGCCACGACCTCGG

TGTCCAGCTGGCCCGGGCCCTGGCCTGGTTCTCAAGTGTTTCCTAGACAGAGAGGCACCT
3001 ---------+---------+---------+---------+---------+---------+ 3060
ACAGGTCGACCGGGCCCGGGACCGGACCAAGAGTTCACAAAGGATCTGTCTCTCCGTGGA

GGGTCAGTATTAGTCTATTTATCAGAGGTGTAAATAATCTATGTATAGTTTTTCTCCTTT
3061 ---------+---------+---------+---------+---------+---------+ 3120
CCCAGTCATAATCAGATAAATAGTCTCCACATTTATTAGATACATATCAAAAAGAGGAAA

TAGATTATTTTGTATTTGTTTAAAAGAAGTTTTGTCAAAATACAAAAATATAAAGAAATG
3121 ---------+---------+---------+---------+---------+---------+ 3180
ATCTAATAAAACATAAACAAATTTTCTTCAAAACAGTTTTATGTTTTTATATTTCTTTAC

ACTGAAAGTTGTTGACAGGGTTTTTAAGAAATAATTATTCTAATTGTTTTTGTTTGTTTG
3181 ---------+---------+---------+---------+---------+---------+ 3240
TGACTTTCAACAACTGTCCCAAAAATTCTTTATTAATAAGATTAACAAAAACAAACAAAC

TTTTTGCCTTGTAAACTAGCGCCAAGGAACTGCAGCAAATAAACTCCAACTCTGCCCAAG
3241 ---------+---------+---------+---------+---------+---------+ 3300
AAAAACGGAACATTTGATCGCGGTTCCTTGACGTCGTTTATTTGAGGTTGAGACGGGTTC

CAAAAAAAAAA
3301 ---------+- 3311
GTTTTTTTTTT

## Patentansprüche

1. Verwendung eines Cytohesin-PH-Peptids zur Regulation der T-Lymphozyten-Aktivierung.

2. Verwendung eines Cytohesin-PH-Peptids gemäß Anspruch 1 mit der Aminosäuresequenz gemäß Tabelle 2 oder Teilen davon.

3. Verwendung eines Cytohesin-PH-Peptids gemäß Anspruch 1 mit der Aminosäuresequenz von Position 258 bis 398 gemäß Tabelle 2.

4. Verwendung einer DNA, kodierend für ein Cytohesin-PH-Peptid gemäß Anspruch 1 bis 3, oder Teile davon, zur Expression des Cytohesin-PH-Peptids.

5. Verwendung einer DNA nach Anspruch 4 mit der Nukleotidsequenz von Position 841 bis 1263 gemäß Tabelle 2.

6. Verwendung einer DNA nach Anspruch 4, dadurch gekennzeichnet, daß sie unter stringenten Bedingungen mit der DNA gemäß Tabelle 2 hybridisiert.

7. Verwendung einer DNA gemäß Anspruch 4, dadurch gekennzeichnet, daß deren Sequenz in bezug auf die Sequenz der DNA gemäß Anspruch 4 entsprechend dem Wesen des genetischen Codes degeneriert ist.

8. Verwendung eines Vektors enthaltend eine DNA nach einem der Ansprüche 4 bis 7 zur Expression eines Cytohesin-PH-Peptids.

9. Verwendung einer Wirtszelle enthaltend einen Vektor gemäß Anspruch 8.

10. Verwendung eines Cytohesin-PH-Peptids gemäß Anspruch 1 bis 3 zur Beeinflussung von Entzündungen.

11. Verwendung eines Cytohesin-PH-Peptids gemäß Anspruch 1 bis 3 zur Verbesserung der Wundheilung.

12. Verwendung eines Cytohesin-PH-Peptids gemäß Anspruch 1 bis 3 zur Suppression des Immunsystems.

13. Verwendung eines Cytohesin-PH-Peptids gemäß Anspruch 1 bis 3 zur Suppression des Immunsystems bei Organtransplantationen.

14. Verwendung eines Cytohesin-PH-Peptids gemäß Anspruch 1 bis 3 zur Verhinderung der Metastasierung bei hämatopoietischen Tumoren.

15. Verwendung eines Cytohesin-PH-Peptids gemäß Anspruch 1 bis 3 zur Behandlung von Arteriosklerose.

16. Verfahren zur Herstellung eines Cytohesin-PH-Peptids gemäß Anspruch 1 bis 3, dadurch gekennzeichnet, daß
a) eine Wirtszelle gemäß Anspruch 9 kultiviert wird und
b) das Cytohesin-PH-Peptid isoliert wird.

17. Arzneimittel enthaltend ein Cytohesin-PH-Peptid gemäß Anspruch 1 bis 3 und einen physiologisch annehmbaren Träger sowie gegebenenfalls geeignete Zusatz- und Hilfsstoffe.

18. Testkit, das mindestens ein Cytohesin-PH-Peptid oder ein Cytohesin-2-Peptid gemäß einem der Ansprüche 1 bis 3 umfaßt.

19. Testkit gemäß Anspruch 18, das zum Wirkstoff-Screening verwendet werden kann.

20. Verfahren zur Bewertung der Wirkung einer Prüfsubstanz auf die Integrin-Aktivität, das die folgenden Schritte umfaßt: die Prüfsubstanz wird mit Cytohesin-PH gemäß einem der Ansprüche 1 bis 3 in Kontakt gebracht; und die Wirkungen der Prüfsubstanz auf die Aktivität von Cytohesin-PH werden bestimmt.

21. Verfahren gemäß Anspruch 20, bei dem die Prüfsubstanz an eine unlösliche Matrix gebunden ist und Cytohesin-PH markiert wird.

22. Verfahren gemäß Anspruch 20, bei dem Cytohesin-PH an eine unlösliche Matrix gebunden ist und die Prüfsubstanz markiert wird.

23. Verfahren zur Bewertung der Wirkungen einer Verbindung auf die Integrin-Aktivität, das die folgenden Schritte umfaßt:
Züchten von Zellen einer Testgruppe und einer Kontrollgruppe, die die Fähigkeit besitzen, an einem Substrat zu haften,wobei die Testgruppe in Gegenwart einer Prüfsubstanz und die Kontrollgruppe in Abwesenheit einer Prüfsubstanz kultiviert wird;
Einleiten der Expression von Cytohesin-PH gemäß einem der Ansprüche 1 bis 3 in den Testzellen und den Kontrollzellen; und Vergleich des Ausmasses des Haftungsverlustes in der Testgruppe und in der Kontrollgruppe.

24. Cytohesin-2 Peptid mit der Aminosäuresequenz gemäß Figur 1B.

25. Verwendung eines Cytohesin-2-Peptids gemäß Anspruch 24 mit der von der cts 18.1-cDNA oder degenerierten Varianten davon kodierten Aminosäuresequenz zur Regulation der T-Lymphozyten-Aktivierung.

26. DNA, kodierend für ein Cytohesin-2 Peptid gemäß Anspruch 24 oder Teile davon.

27. DNA, kodierend für ein Cytohesin-2 Peptid gemäß Anspruch 24 oder Teile davon, dadurch gekennzeichnet, daß deren Sequenz in bezug auf die Sequenz der DNA gemäß Anspruch 4 entsprechend dem Wesen des genetischen Codes degeneriert ist.

28. Verwendung einer DNA gemäß Anspruch 27, kodierend für ein Cytohesin-2 Peptid gemäß Anspruch 24, zur Expression des Cytohesin-2 Peptids.

29. Verwendung eines Cytohesin-2 Peptids gemäß Figur 1B zur Verwendung gemäß einem oder mehreren der Ansprüche 10 bis 15.

30. Assaysystem gemäß einem oder mehreren der Ansprüche 18 bis 23, enthaltend ein Cytohesin-2 Peptid, gemäß Anspruch 24.

31. Arzneimittel, enthaltend ein Cytohesin-2 Peptid gemäß Anspruch 24 und einen physiologisch annehmbaren Träger sowie gegebenenfalls geeignete Zusatz- und Hilfsstoffe.


SEQUENZPROTOKOLL

(1) ALLGEMEINE INFORMATION:

(i) ANMELDER:
(A) NAME: Hoechst Aktiengesellschaft
(B) STRASSE: -
(C) ORT: Frankfurt am Main
(D) BUNDESLAND: -
(E) LAND: Deutschland
(F) POSTLEITZAHL: 65926
(G) TELEPHON: 069-305-3005
(H) TELEFAX: 069-35-7175
(I) TELEX: 4 1 234 700 ho d

(ii) ANMELDETITEL: Verwendung von Cytohesin-PH-Peptiden zur
Beeinflussung der Adhaesionsfaehigkeit von Integrinen

(iii) ANZAHL DER SEQUENZEN: 10

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..30

(xi) SEQUENZBESCHREIBUNG: SEQ ID NO: 1:

CGCGGGACGC GTGCTCTGAT CCACCTGAGC 30

(2) INFORMATION ZU SEQ ID NO: 2:

(i) SEQUENZ CHARAKTERISTIKA:
(A) LÄNGE: 36 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..36

(xi) SEQUENZBESCHREIBUNG: SEQ ID NO: 2:

CGCGGGGCGG CCGCTTTAAC TCTCAGCAAA CTTGGG 36

(2) INFORMATION ZU SEQ ID NO: 3:

(i) SEQUENZ CHARAKTERISTIKA:
(A) LÄNGE: 39 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..39

(xi) SEQUENZBESCHREIBUNG: SEQ ID NO: 3:

CGCGGGACGC GTATGGAGGA GGACGACAGC TACGTTCCC 39

(2) INFORMATION ZU SEQ ID NO: 4:

(i) SEQUENZ CHARAKTERISTIKA:
(A) LÄNGE: 42 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..42

(xi) SEQUENZBESCHREIBUNG: SEQ ID NO: 4:

CGCGGGGCGG CCGCTTTAGT GTCGCTTCGT GGAGGAGACC TT 42

(2) INFORMATION ZU SEQ ID NO: 5:

(i) SEQUENZ CHARAKTERISTIKA:
(A) LÄNGE: 39 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..39

(xi) SEQUENZBESCHREIBUNG: SEQ ID NO: 5:

GCGGGGACGC GTACCATGGC TAATGAAATT GAAAACCTG 39

(2) INFORMATION ZU SEQ ID NO: 6:

(i) SEQUENZ CHARAKTERISTIKA:
(A) LÄNGE: 42 Basenpaare

(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..42

(xi) SEQUENZBESCHREIBUNG: SEQ ID NO: 6:

GCGGGGGCGG CCGCTTTAGA AAGTGTGAGT GAGGTCATTC CC 42

(2) INFORMATION ZU SEQ ID NO: 7:

(i) SEQUENZ CHARAKTERISTIKA:
(A) LÄNGE: 45 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..45

(xi) SEQUENZBESCHREIBUNG: SEQ ID NO: 7:

CGCGGGACGC GTACCATGGG TTTCAATCCA GACCGAGAAG GCTGG 45

(2) INFORMATION ZU SEQ ID NO: 8:

(i) SEQUENZ CHARAKTERISTIKA:
(A) LÄNGE: 42 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..42

(xi) SEQUENZBESCHREIBUNG: SEQ ID NO: 8:

CGCGGGGCGG CCGCTTTAGT GTCGCTTCGT GGAGGAGACC TT 42

(2) INFORMATION ZU SEQ ID NO: 9:

(i) SEQUENZ CHARAKTERISTIKA:
(A) LÄNGE: 3251 Basenpaare
(B) ART: Nukleinsäure
(C) STRANGFORM: Doppel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..3251

(xi) SEQUENZBESCHREIBUNG: SEQ ID NO: 9:

GCGAGCGGGG GCGCGGGTGG CGCGGCGGGA CGCGAGCGGC GAGCCGGAGC GCGAGCCCGC 60

TCCCGCACCA TGGAGGAGGA CGACAGCTAC GTTCCCAGTG ACCTGACAGC AGAGGAGCGT 120

CAAGAACTGG AGAACATCCG ACGGAGAAAA CAGGAGCTGC TGGCTGACAT TCAGAGGCTG 180

AAGGATGAGA TAGCAGAAGT AGCTAATGAA ATTGAAAACC TGGGATCCAC AGAGGAAAGG 240

AAAAACATGC AGAGGAACAA ACAGGTAGCC ATGGGCAGGA AAAAATTTAA TATGGACCCT 300

AAAAAGGGGA TCCAGTTCTT AATAGAGAAC GACCTCCTGA AGAACACTTG TGAAGACATT 360

GCCCAGTTCT TATATAAAGG CGAAGGGCTC AACAAGACAG CCATCGGCGA CTACCTAGGG 420

GAGAGAGATG AGTTTAATAT CCAGGTTCTT CATGCATTTG TGGAGCTGCA TGAGTTCACT 480

GATCTTAATC TCGTCCAGGC ACTACGGCAG TTCCTGTGGA GCTTCCGGCT ACCCGGAGAG 540

GCCCAGAAGA TCGACCGGAT GATGGAGGCG TTTGCCCAGC GATATTGTCA GTGCAATAAT 600

GGCGTGTTCC AGTCCACGGA TACTTGTTAC GTCCTCTCCT TTGCCATCAT CATGTTGAAC 660

ACCAGTCTGC ACAACCCCAA TGTCAAAGAT AAGCCCACTG TGGAGAGGTT CATTGCCATG 720

AACCGAGGCA TCAATGATGG GGGAGACCTG CCGGAGGAGC TCCTCCGGAA TCTCTATGAG 780

AGCATAAAAA ATGAACCCTT TAAAATCCCA GAAGACGACG GGAATGACCT CACTCACACT 840

TTCTTCAATC CAGACCGAGA AGGCTGGCTA TTGAAACTCG GAGGTGGCAG GGTAAAGACT 900

TGGAAGAGAC GCTGGTTCAT TCTGACTGAC AACTGCCTTT ACTACTTTGA GTATACCACG 960

GATAAGGAGC CCCGTGGAAT CATCCCTTTA GAGAATCTGA GTATCCGGGA AGTGGAGGAC 1020

TCCAAAAAAC CAAACTGCTT TGAGCTTTAT ATCCCCGACA ATAAAGACCA AGTTATCAAG 1080

GCCTGCAAGA CCGAGGCTGA CGGGCGGGTG GTGGAGGGGA ACCACACTGT TTACCGGATC 1140

TCAGCTCCGA CGCCCGAGGA GAAGGAGGAG TGGATTAAGT GCATTAAAGC AGCCATCAGC 1200

AGGGACCCTT TCTACGAAAT GCTCGCAGCA CGGAAAAAGA AGGTCTCCTC CACGAAGCGA 1260

CACTGAGCGT GCAGCCAAGG GCGTTGGTCT GCGGGGGCCT TGGAGCTCCT GCTCTTCTCC 1320

CGCACCTCCA TGGATGCACT GCTGCCGAGC AGAGCGTCCT CTGCCAGGCC CCGCCCTGGA 1380

TTCCTAGAGA CTAGCTTCAG CTTTTGCTAT TTTTTTTAAG TGGGAGAAGG GTGGGCAGTT 1440

ATCACTGGGG AAGAGAGGAC CGGCCACCTG TCCAGCATGG GCTCCAGAGC CTTCCTCTCT 1500

CACAGGGCAG AGCTCTTGTC GGCAGGGCAG CCTCCTGGCC AGTTTCTCTG CTCAGTGTTC 1560

TGGTAGCAGA GCTCAGAGCC AACTGTTTAC CTCTTGGTTG TCCCCGTGAA GAAGCCTTCA 1620

AACCCTGCAC CATAAATACA TGTGTCCATA TATTATTATA TGTTAAGAGA AAAAGGTGGA 1680

AAGGAAGAGA AGCCACATAC TATAAAGATC TATTTTTTTT TTTTAAGAGA GAACGTAGGG 1740

CTGTTCAGGT GCATTCTGCC CTGGCTGCGC TGGGGAGCTT CTCCCTGGAG AAGAGCACCT 1800

GGGGCTGCGG CCAAGGGGCA TCAGCCTGGG CCCGCGGCAG GGCCTGGCCT GCCTCTCCTG 1860

TGCTGTGGGA GCTCGCTGCC TGGTGCTTGT CTTGGCGAGA TGGACAGGTG AGGTCGAGGA 1920

CGCAGAGGGC AGAGGCCCAG TGGAGCCTCA GACGGCACAG TCAGAGTCGG GGGCCTGCCT 1980

GGCCGGGGTC GCAGTCGGCA GCAGCGTGCA GTCCGGCATC TCCCGCGGAT GCTTTTCCAT 2040

CCCAAGTGCC TGCGGAGCCC GAGGAGAGGA GAGAGCTGAC TGGACGCTTA CGTTATTTTC 2100

CTCCTTCAGA ATCCAAGTTC TTGTTGGGCT TTAAAGTAGA AAGTCAGCAT TTTCCTTGAG 2160

CTAAATACCT AATAACCAAA ACTGTGAGGA AGGTTATCGG GACAGAGGTT CCGGATAACC 2220

TGTTTCATTT TGGGTTTTCT TCCTCTTCCC CAGACTCCAG TCCTCGTTCT AGAGGAAGGA 2280

GTAGGACTTC CCCGATCCCC GTAGCTTCAG CTTTTTCTGC CTCAAAACCA GCCCTAACTG 2340

GACTACTCTG GATGCATTTT GTGGTGGGCC CCCTAGAGGG AAGATGGGCC TTTATCTGCT 2400

CCGTGGGGTG CACTGGAGTG AGGGGGGTGG CCGGGCTGCC TCTCGCATCT CTGTCTTCCC 2460

CTGCAGGCGC TGTGTGAGCT GGCCCTGCCC CTCCTCATTA CAGTATGAAG GGAGCCGTGA 2520

CACGCAGCAT TTTCCTGCCG TTCTCTCAGG GACTCTCAGG GCAGCTCCTG CCACTCCGCC 2580

AGGGCCAGCA TGCCAGTCCA GGCAGAGCAG GTGGCTGGCT GTCTGGCCGT CTCGCCCCGC 2640

CCCTCCACAG GACCCTGGAC CAGGGCGGTG CAGGGCGCAG CCCCGAGGAG GCAGGTGGAG 2700

GAGCTGCGGG TTTTCACAGG GCCGCGTCGC CACGGCTCCT CTGATCCTTT AGGGTTGGCG 2760

AGCATCTCTG GAAATAGCTT TTGCAGAGGA GTGGTGGGAG GAATAGAGGG GGACAGTCTG 2820

TCACCTCCCT CCCCGCCACT TTGTGTAGAT CCTACCTGGA GGGAATGGCT TTAGGCACTT 2880

TTGTGCCAGA GCTTGTGAGG GTGACAGAAG AGGGTCCAGG CTGGAAACCT GAACTTTCTG 2940

GGTGGGAGAA CCAGGTGGTG CCTGCCGAGG TCTGGGCGTG TTTGGGCCGG TGCTGGAGCC 3000

TGTCCAGCTG GCCCGGGCCC TGGCCTGGTT CTCAAGTGTT TCCTAGACAG AGAGGCACCT 3060

GGGTCAGTAT TAGTCTATTT ATCAGAGGTG TAAATAATCT ATGTATAGTT TTTCTCCTTT 3120

TAGATTATTT TGTATTTGTT TAAAAGAAGT TTTGTCAAAA TACAAAAATA TAAAGAAATG 3180

ACTGAAAGTT GTTGACAGGG TTTTTAAGAA ATAATTATTC TAATTGTTTT TGTTTGTTTG 3240

TTTTTGCCTT GTAAACTAGC GCCAAGGAAC TGCAGCAAAT AAACTCCAAC TCTGCCCAAG 3300

CAAAAAAAAA A 3311

(2) INFORMATION ZU SEQ ID NO: 10:

(i) SEQUENZ CHARAKTERISTIKA:
(A) LÄNGE: 398 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..398

(xi) SEQUENZBESCHREIBUNG: SEQ ID NO: 10:

Met Glu Glu Asp Asp Ser Tyr Val Pro Ser Asp Leu Thr Ala Glu Glu
1               5                   10                  15

Arg Gln Glu Leu Glu Asn Ile Arg Arg Arg Lys Gln Glu Leu Leu Ala
            20                  25                  30

Asp Ile Gln Arg Leu Lys Asp Glu Ile Ala Glu Val Ala Asn Glu Ile
        35                  40                  45

Glu Asn Leu Gly Ser Thr Glu Glu Arg Lys Asn Met Gln Arg Asn Lys
    50                  55                  60

Gln Val Ala Met Gly Arg Lys Lys Phe Asn Met Asp Pro Lys Lys Gly
65                  70                  75                  80

Ile Gln Phe Leu Ile Glu Asn Asp Leu Leu Lys Asn Thr Cys Glu Asp
                85                  90                  95

Ile Ala Gln Phe Leu Tyr Lys Gly Glu Gly Leu Asn Lys Thr Ala Ile
            100                 105                 110

Gly Asp Tyr Leu Gly Glu Arg Asp Glu Phe Asn Ile Gln Val Leu His
        115                 120                 125

Ala Phe Val Glu Leu His Glu Phe Thr Asp Leu Asn Leu Val Gln Ala
    130                 135                 140

Leu Arg Gln Phe Leu Trp Ser Phe Arg Leu Pro Gly Glu Ala Gln Lys
145                 150                 155                 160

Ile Asp Arg Met Met Glu Ala Phe Ala Gln Arg Tyr Cys Gln Cys Asn
                165                 170                 175

Asn Gly Val Phe Gln Ser Thr Asp Thr Cys Tyr Val Leu Ser Phe Ala
            180                 185                 190

Ile Ile Met Leu Asn Thr Ser Leu His Asn Pro Asn Val Lys Asp Lys
        195                 200                 205

Pro Thr Val Glu Arg Phe Ile Ala Met Asn Arg Gly Ile Asn Asp Gly
    210                 215                 220

Gly Asp Leu Pro Glu Glu Leu Leu Arg Asn Leu Tyr Glu Ser Ile Lys
225                 230                 235                 240

Asn Glu Pro Phe Lys Ile Pro Glu Asp Asp Gly Asn Asp Leu Thr His
                245                 250                 255

Thr Phe Phe Asn Pro Asp Arg Glu Gly Trp Leu Leu Lys Leu Gly Gly
            260                 265                 270

Gly Arg Val Lys Thr Trp Lys Arg Arg Trp Phe Ile Leu Thr Asp Asn
        275                 280                 285

Cys Leu Tyr Tyr Phe Glu Tyr Thr Thr Asp Lys Glu Pro Arg Gly Ile
    290                 295                 300

Ile Pro Leu Glu Asn Leu Ser Ile Arg Glu Val Glu Asp Ser Lys Lys
305                 310                 315                 320

Pro Asn Cys Phe Glu Leu Tyr Ile Pro Asp Asn Lys Asp Gln Val Ile
                325                 330                 335

Lys Ala Cys Lys Thr Glu Ala Asp Gly Arg Val Val Glu Gly Asn His
            340                 345                 350

Thr Val Tyr Arg Ile Ser Ala Pro Thr Pro Glu Glu Lys Glu Glu Trp
        355                 360                 365

Ile Lys Cys Ile Lys Ala Ala Ile Ser Arg Asp Pro Phe Tyr Glu Met
    370                 375                 380

Leu Ala Ala Arg Lys Lys Lys Val Ser Ser Thr Lys Arg His
385                 390                 395